# EUROPEAN PATENT SPECIFICATION

(11) **EP 1 682 057 B1**
(45) Date of publication and mention of the grant of the patent: **11.03.2009**
(21) Application number: 04783134.2
(22) Date of filing: 02.09.2004
(51) Int. Cl.: A61F 9/007

(54) **PHACOEMULSIFICATION NEEDLE**
PHAKOEMULSIFIKATIONSNADEL
AIGUILLE POUR PHACOEMULSIFICATION

(30) Priority: 17.09.2003 US 664837
(43) Date of publication of application: 26.07.2006
(73) Proprietor: BAUSCH & LOMB INCORPORATED, Rochester, NY 14604-2701 (US)
(72) Inventor: PERKINS, James, T., St. Charles, MO 63304 (US); FERREIRA, Paul, J., Stamford, CT 06905 (US)
(74) Representative: Vossius & Partner
(86) International application number: PCT/US2004/028780
(87) International publication number: WO 2005/032439

(56) References cited:
- US-A- 4 816 018
- US-A- 5 464 389
- US-A- 5 989 209

## Description

### Background of the Invention

### 1. Field of the Invention

The present invention is directed to a phacoemulsification needle for use in ophthalmic surgery, particularly in cataract surgery. More specifically, the present invention is directed to a phaco needle having at least two (2) distinct inner diameters.

### 2. Description of Related Art

Phacoemulsification (phaco) needles are well-known in ophthalmic surgery for removing cataracts. Typically, a phaco needle has an inner diameter through which emulsified or chopped-up particles of a patient's cataractus lens are aspirated through the needle and a handpiece to a collection bag or reservoir. It has become common practice for the phaco needle to be vibrated with ultrasonic energy transmitted from a handpiece connected to the needle. This ultrasonic energy acts to break-up tissue, which tissue is then aspirated through the inner diameter of the phaco needle.

Different phaco needle constructions have been developed in the prior art to assist in breaking up a patient's cataract. For instance, Parisi in U.S. Patent 4,816,018 discloses a phaco needle tip having a large inner diameter bore at a distal end of the phaco tip compared with a second smaller bore through out the remainder of the phaco needle. Such a larger inner diameter at the distal end of the phaco needle is provided to concentrate the ultrasonic energy within the larger bore of the needle to emulsify the bodily tissues more effectively then that accomplished by a constant diameter bored needle.

Likewise, U.S. Patent 5,451,229 to Geuder, et al. teaches a stepped distal end of a phaco needle, wherein each successively smaller bore helps to further fragment a cataract tissue for aspiration through the needle. Another example of a multiple diameter phaco needle tip is Sutton, et al. in U.S. Patent 6,533,750 that teaches the use of a conically shaped phaco tip for concentrating the ultrasonic energy for emulsifying a cataract tissue.

These prior art phaco needles with large diameter distal tips are effective at emulsifying tissue within the inner diameter of the tip but yet can have a repulsive effect that tends to push large lens fragments away from the phaco tip and does not allow the phaco needle to effectively bore deep into large fragments of the lens, particularly at lower vacuum settings. This characteristic of these needles makes the use of the common cataract surgery procedure called a chopping method to be more difficult to perform. Therefore, it would be desirable to have a needle that provides some of the advantages of the larger bore phaco tips but yet allows the phaco needle to more easily bore deep into large lens fragments.

This problem is solved by the invention as defined in claim 1. Preferred embodiments are described in the dependent claims.

### Brief Description of Drawings

FIG. 1 is a perspective view of a phaco needle in accordance with the present invention;
FIG. 2 is a cut away perspective view of a phaco needle in accordance with the present invention attached to a surgical handpiece;
FIG. 3 is a chart comparing a standard phaco needle to the present invention at varying vacuum levels;
FIG. 4 is a cut away elevation view of a phaco needle in accordance with the present invention;
FIG. 5 is a partial cut away elevation of an alternative embodiment in accordance with the present invention; and
FIG. 6 is a partial cut away elevation of yet another alternative embodiment in accordance with the present invention.

### Detailed Description of the Preferred Embodiment

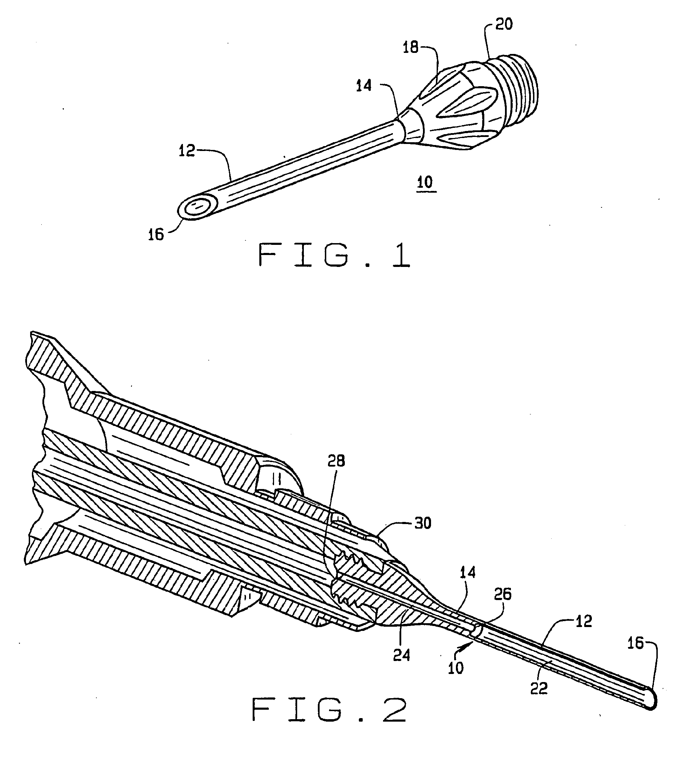

FIG. 1 shows a phaco needle or cannula 10 in accordance with the present invention. Phaco needle 10 includes a needle 12 having a proximal end 14 and a distal end 16. Proximal end 14 is attached to a hub 18, which includes threads 20 for engagement with a surgical instrument shown below. Attachment structures other than threads 20 may be used to attach cannula 10 to a surgical instrument.

FIG. 2 shows a cut away perspective view of the needle 10. Phaco cannula 10 includes the needle 12 having a first and a second diameter 22 and 24, respectively. As can be seen, the first inner diameter 22 is larger than the second inner diameter 24. In addition, a transition 26 from the first inner diameter 22 to the second inner diameter 24 is closer to the proximal end 14 than to the distal end 16. Another way to view cannula 10 is that it has an elongated needle 12 having a distal end 16 and a proximal end 28 structured for attachment to a surgical instrument 30, such as by threads 20 as shown in FIG. 2. In this view, it is still important that the transition 26 be closer to proximal end 28 than to distal end 16.

One of the main advantages of the present invention is to provide a reduced inner diameter that allows a surgeon to operate at a higher vacuum range, but yet with a lower flow rate. This combination of higher vacuum range and lower flow rate than is possible in some prior art needles, allows the surgeon to bore deep into a cataract and hold the cataract while a second instrument is used to segment the cataract (i.e. chopping). This deep bore is more easily achieved than in the prior art because of the transition being moved from near the distal end to near the proximal end of the cannula. This allows a surgeon to have superior vacuum holding power but yet have a low flow rate so that if problems were to occur, the surgeon has time to react before irreversible damage can occur.

Yet another advantage and benefit of the present invention is that the transition 26 from a large inner diameter to a small inner diameter provides a higher degree of ultrasonic displacement energy than if the larger inner diameter were toward the distal end 16. Placing the transition 26 as close to the surgical handpiece 30 as possible ensures that the aspirated tissue is completely emulsified and minimizes any possibility of clogging in the aspiration tubing (not shown). There is more ultrasonic energy at the transition from large to small diameter as the transition is moved closer to the source of the ultrasonic energy - the handpiece.

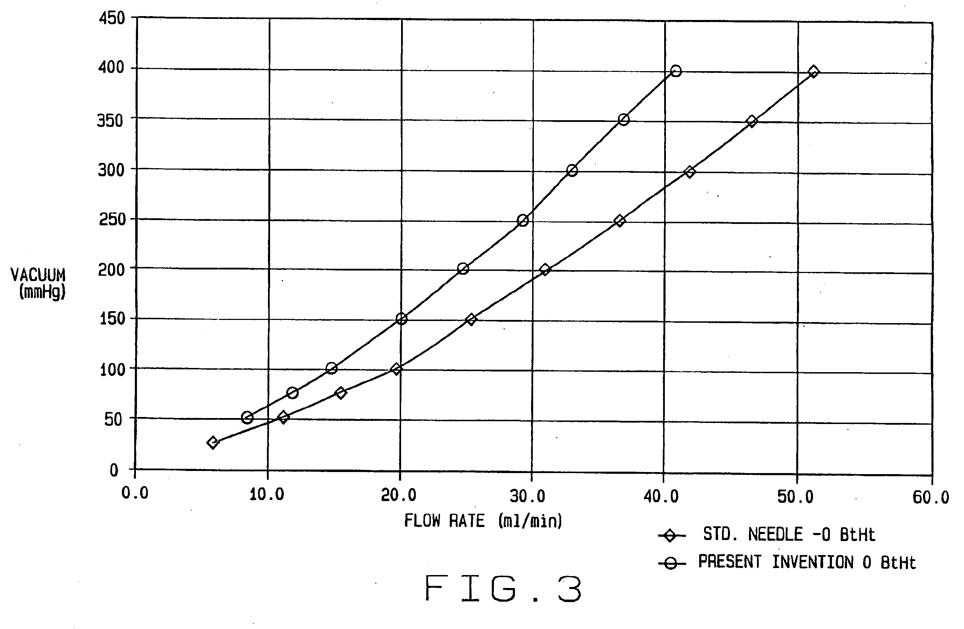

FIG. 3 shows a graph of vacuum in millimeters of mercury versus flow rate in milliliters per minute for both a standard phaco needle and the needle of the present invention at a zero (0) bottle height. As can be seen, the present invention provides for higher vacuum levels at any given flow rate as compared to a standard needle. This provides again, as stated above, the surgeon with greater holding power for the cataract at lower flow rates, which should ensure quicker and safer surgical procedures.

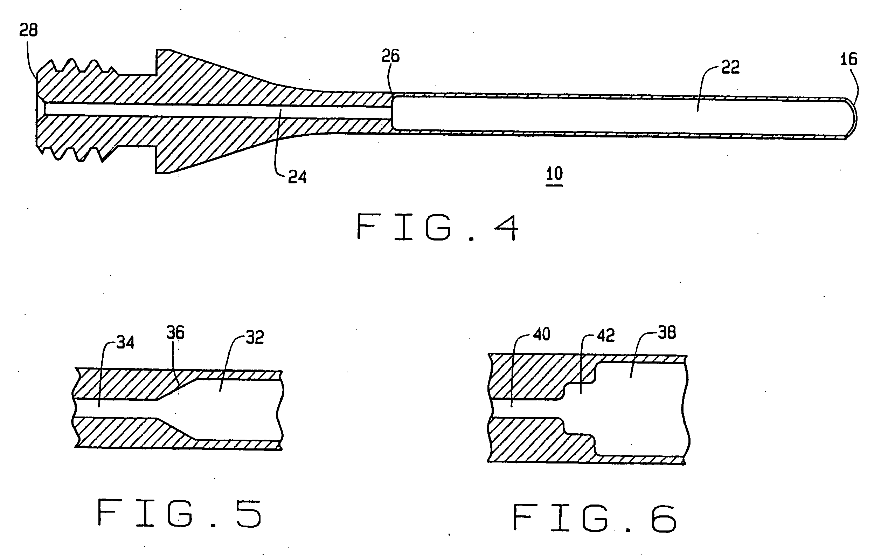

FIG. 4 shows phaco needle 10 including a first bore or inner diameter 22 extending from the distal end 16 toward the proximal end 28, a second bore or inner diameter 24 within the cannula 10 extends from the proximal end 28 to the first bore 22. As can be seen, the second bore 24 has a smaller diameter than the first bore. It is also preferable that second bore 24 is of sufficient length to provide a desired pressure drop during use across the length of the second bore 24. This pressure drop enables the user to use higher vacuum levels and still maintain relatively low flow rates. Also, it is preferred that the intersection or transition 26 of the first and second bores 22 and 24 is nearer the proximal end 28 than to the distal end 16.

As shown in FIG. 4, the transition 26 may simply be a radius. However, the transition between first bore 22 and second bore 24 may take on other configurations, such as those shown in FIGs. 5 and 6. FIG. 5 shows a partial needle depicting the transition between a first bore 32 and a second bore 34 having a conical transition 36. Likewise, FIG. 6 shows a transition from a first bore 38 to a second bore 40 having a stepped transition 42. Obviously multiple stepped transitions may also be used. Depending on the application and a particular surgeon's preference, the various transitions 26, 36, 42, or some other configuration may be used within a needle in accordance with the present invention.

## Claims

1. A phacoemulsification device comprising:
an elongated needle having proximal and distal ends, wherein the proximal end is structured for attachment to a surgical instrument;
the needle having a bore having a section with a first and a section with a second inner diameter wherein the first inner diameter is larger than the second inner diameter and wherein the first inner diameter extends from the distal end towards the proximal end, **characterized in that** a transition from the first inner diameter to the second inner diameter is closer to the proximal end than to the distal end.

2. The device of claim 1, wherein the transition includes a radius.

3. The device of claim 1, wherein the transition includes a conical surface connecting the first and second inner diameter sections.

4. The device of claim 1, wherein the transition includes at least one section with an additional inner diameter stepped between the first and second inner diameter sections wherein the additional inner diameter is smaller than the first inner diameter and larger than the second inner diameter.

5. The device of any one of the preceding claims, wherein the device is either a needle or cannula.

6. The device of any one of the preceding claims, further comprising a hub for engaging the device with the surgical instrument.

## Patentansprüche

1. Phakoemulsifikationsvorrichtung mit:
einer länglichen Nadel mit einem proximalen und einem distalen Ende, wobei das proximale Ende zur Befestigung an einem chirurgischen Instrument strukturiert ist;
wobei die Nadel eine Bohrung hat, die ein Teilstück mit einem ersten und ein Teilstück mit einem zweiten Innendurchmesser hat, wobei
der erste Innendurchmesser größer als der zweite Innendurchmesser ist und wobei sich der erste Innendurchmesser vom distalen Ende zum proximalen Ende erstreckt,
**dadurch gekennzeichnet, daß** ein Übergang vom ersten Innendurchmesser zum zweiten Innendurchmesser näher zum proximalen Ende als zum distalen Ende liegt.

2. Vorrichtung nach Anspruch 1, wobei der Übergang einen Radius aufweist.

3. Vorrichtung nach Anspruch 1, wobei der Übergang eine Kegelfläche aufweist, die die Teilstücke mit dem ersten und zweiten Innendurchmesser verbindet.

4. Vorrichtung nach Anspruch 1, wobei der Übergang mindestens ein Teilstück mit einem zusätzlichen Innendurchmesser aufweist, der zwischen den Teilstücken mit dem ersten und zweiten Innendurchmesser abgestuft ist, wobei der zusätzliche Innendurchmesser kleiner als der erste Innendurchmesser und größer als der zweite Innendurchmesser ist.

5. Vorrichtung nach einem der vorstehenden Ansprüche, wobei die Vorrichtung eine Nadel oder Kanüle ist.

6. Vorrichtung nach einem der vorstehenden Ansprüche, ferner mit einer Nabe zum Herstellen eines Eingriffs der Vorrichtung mit dem chirurgischen Instrument.

## Revendications

1. Dispositif de phacoémulsification comportant :
une aiguille allongée ayant une extrémité proximale et une extrémité distale, dans laquelle l'extrémité proximale est conçue pour être montée sur un instrument chirurgical,
l'aiguille ayant un canal dont une section présente un premier diamètre intérieur et une seconde section présente un second diamètre intérieur, le premier diamètre intérieur étant plus grand que le second diamètre intérieur et le premier diamètre intérieur s'étendant depuis l'extrémité distale vers l'extrémité proximale **caractérisé en ce que** la transition du premier diamètre intérieur au second diamètre intérieur est plus près de l'extrémité proximale que de l'extrémité distale.

2. Dispositif selon la revendication 1 dans lequel la transition comporte un rayon.

3. Dispositif selon la revendication 1 dans lequel la transition comporte une surface conique reliant les sections de premier diamètre intérieur et de second diamètre intérieur.

4. Dispositif selon la revendication 1 dans lequel la transition comporte au moins une section avec un diamètre intérieur supplémentaire intercalée entre les sections de premier diamètre intérieur et de second diamètre intérieur, le diamètre intérieur supplémentaire étant plus petit que le premier diamètre intérieur et plus grand que le second diamètre intérieur.

5. Dispositif selon l'une quelconque des revendications précédentes, dans lequel dispositif est soit une aiguille soit une canule.

6. Dispositif selon l'une quelconque des revendications précédentes, comportant en outre un mandrin pour mettre le dispositif en prise avec l'instrument chirurgical.
